# EUROPEAN PATENT APPLICATION

(11) **EP 1 161 955 A2**
(43) Date of publication of application: **12.12.2001**
(21) Application number: 01202886.6
(22) Date of filing: 16.05.1996
(51) Int. Cl.: A61K 39/395, A61P 37/06, A61K 35/28, C07K 16/28

(54) **Induction of immunological tolerance for graft transplantation by the use of humanized non-depleting anti-cd4 antibodies in combination with specific donor bone marrow cells**

(30) Priority: 18.05.1995 US 443739
(62) Divisional of application: 96915808.8
(71) Applicant: Ortho Pharmaceutical Corporation, Raritan NJ 08860 (US)
(72) Inventor: Knowles, Robert W., New York, NY 10021 (US); Cavender, Druie E., Flemington, NJ 08822 (US); Thomas, Judith M., Birmingham, AL 35242 (US)
(74) Representative: Mercer, Christopher Paul

(57) **Abstract**

The invention provides a method of inducing immunological tolerance and a method of preventing allograft rejection. In both methods, compositions comprising non-depleting anti-CD4 antibody are administered to a primate subject. Compositions comprising non-depleting antibody and eventually donor bone marrow are further disclosed.

## Description

### BACKGROUND OF THE INVENTION

The present invention relates to the use of non-depleting antibodies to induce immunological tolerance to an antigen, or to inhibit allograft rejection.

The immunosuppressive effects of antibodies against T-lymphocytes are well known. Various monoclonal antibodies which have been shown to induce immunosuppression may be characterized as either depleting antibodies or as non- depleting antibodies. Depleting antibodies are those which cause depletion of cells carrying their target antigen in the recipient. Non-depleting antibodies are those which do not cause depletion of cells carrying their target antigen.

Tolerance induction has been observed in the mouse cardiac allograft model with non-depleting anti-CD4 mAb as described in Darby et al., Transplantation, 54:483-490 September 1992. Prolongation of kidney allograft survival in rhesus monkeys with murine OKT4A is described in Jonker et al., Transplantation, 39:247, 1985. Prolongation of kidney allograft survival in monkeys with a non-depleting antibody, humanized OKT4A is described in Powelson, et al., Transplantation 57:788-793, March 1994. Although Powelson describes prolongation of kidney allograft in primates using a non-depleting anti-CD4 antibody, induction of long term immunological tolerance has not been accomplished in primates. Furthermore, it has been established that tolerance induction in mice is relatively easy to accomplish, and in no way indicates that such tolerance will be possible in higher mammals.

The infusion of donor bone marrow in monkeys treated with rabbit antithymocyte globulin promotes graft tolerance without chronic immunosuppression (Thomas et al., Transplantation 51:198, 1991). Retrospective analysis of long term graft survival in this model has revealed that partial matching between the donor and recipient animals, (one shared allele at the DRB1 major histocompatability gene), increased long term allograft survival following treatment with rabbit anti-thymocyte golbulin and donor bone marrow (Thomas et al., Transplantation 59:245-255, 1995).

### SUMMARY OF THE INVENTION

The present invention provides a method of inducing immunological tolerance to an antigen. Tolerance is conferred by administering to a subject a combination of a non-depleting anti-CD4 antibody and the antigen to which tolerance is desired.

The present invention also provides a method of inhibiting allograft rejections without depleting the patient's T-cell population. Allograft rejection is inhibited by administering to the patient, at the time of the allograft, a non-depleting anti-CD4 antibody. Alternatively, allograft rejection may be prohibited by administering to the patient, at the time of the allograft, a non-depleting anti-CD4 antibody in combination with bone marrow cells from the graft donor.

### BRIEF DESCRIPTION OF THE FIGURES

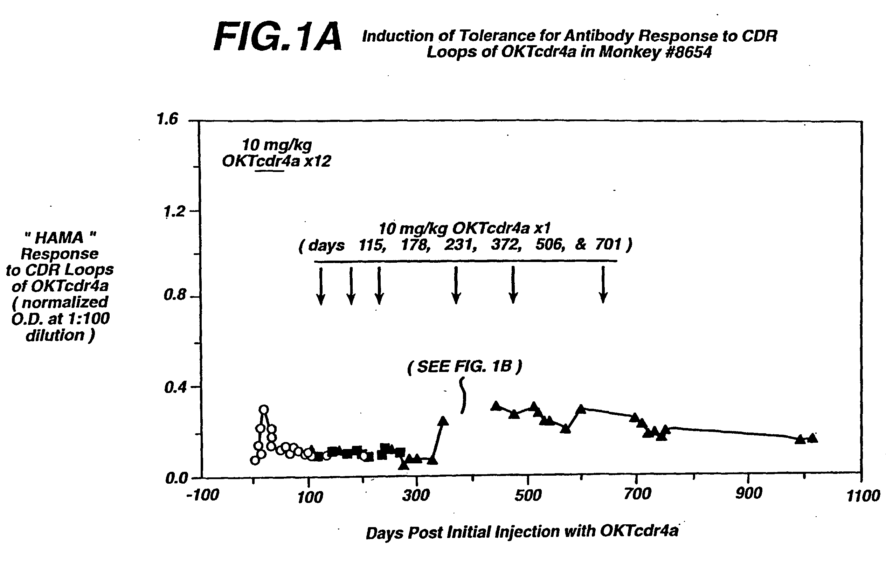

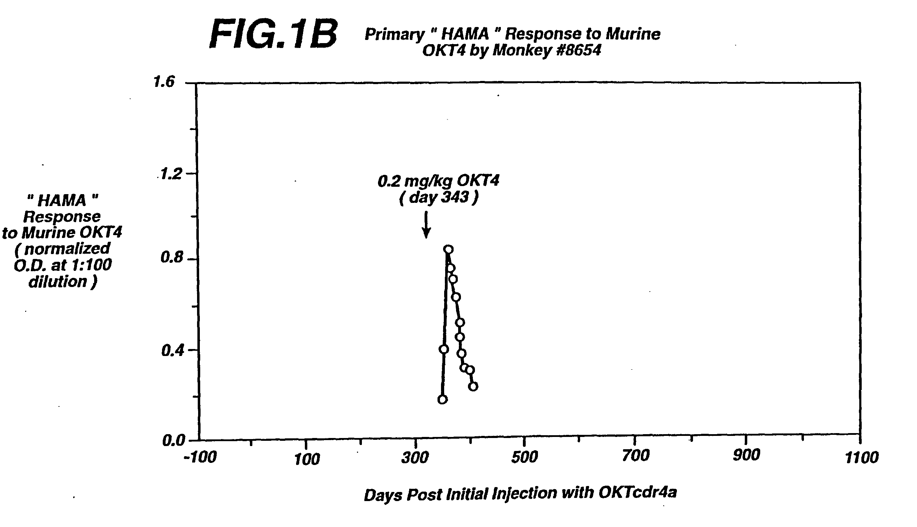

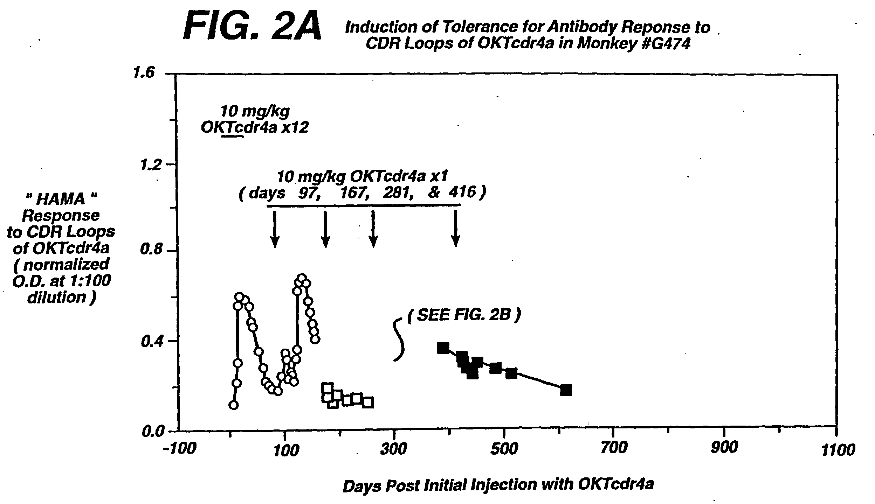

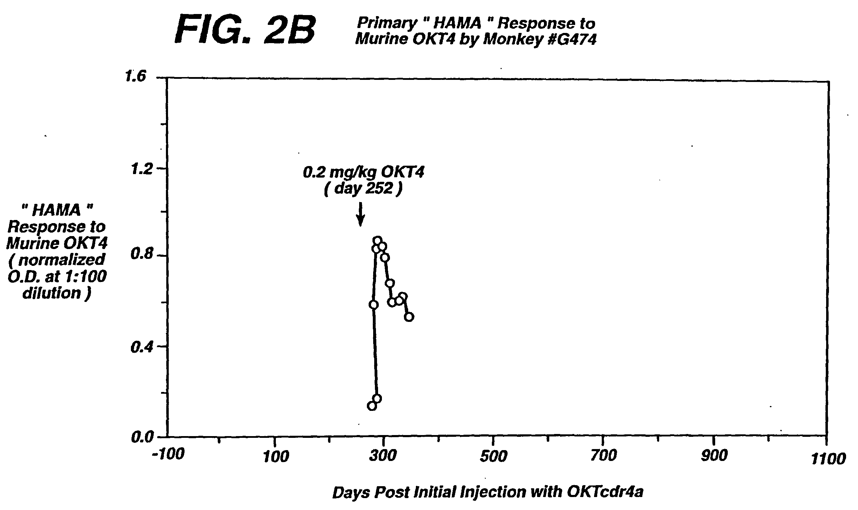

### Brief description of Figures 1A, 1B, 2A, and 2B.

Anti-antibody responses in the two monkeys injected with 10 mg/kg/day of OKTcdr4a for 12 days are shown. An ELISA was designed to detect monkey antibodies that were capable of binding to solid-phase murine OKT4A, which shares its CDR loops in common with OKTcdr4a. The plates could not be coated with OKTcdr4a since any reagent that could detect monkey HAMA would cross-react with OKTcdr4a. Monkeys injected with OKTcdr4a were not expected to produce antibodies to the humanized portions of the molecule due to the high sequence homology of monkey and human immunoglobulins.

Microtiter plates were coated with murine OKT4A. Following the blocking of non-specific adsorption sites with BSA, monkey samples at dilutions >1:100 were added. Binding of monkey HAMA to the plates was detected with a HRPO-conjugated goat anti-monkey IgG (H+L) reagent. A substrate solution containing H2O2 and orthophenylenediamine was then used to develop the reaction.

O.D. readings were normalized to the O.D. reading obtained with a slightly subsaturating dilution of a secondary HAMA-containing sample obtained from another monkey. A 1:100 dilution of a pool of plasma samples from eleven non-injected monkeys was also run on each plate as a negative control. Titers of HAMA were defined as the dilution at which the normalized O.D. value of the peak sample from an injected animal was equal to twice the normalized O.D. reading of the negative control pool. Inhibition experiments on positive samples with soluble mOKT4A or OKTcdr4a demonstrated that the monkey HAMAs were inhibited equally well with either mAb (data not shown), confirming that the murine and humanized antibodies share antigenic epitopes within the CDR loops regions.

Plasma samples were tested by ELISA for the presence of HAMA to OKT4, using the same protocol as the anti-CDR-loops assay described above, except that the samples were tested on plates coated with either mOKT4 or mOKT4A. Since nearly identical O.D. readings were obtained on the two types of plates, the data are presented as the normalized O.D. values from the mOKT4A-coated plate since the raw O.D. readings on that plate could be normalized to the O.D. valueobtained with the positive control run on that plate.

### DETAILED DESCRIPTION OF THE INVENTION

Immunological tolerance is defined as immunological unresponsiveness to challenge with the antigen to which tolerance has been induced. Tolerance is demonstrated when a subject is unresponsive subsequent to challenge with the tolerance-inducing antigen.

The cdr-grafted anti-CD4 antibody designated OKT cdr4a used the present invention is described in International Application Number PCT/GB90/02015, published as International Publication Number WO 91/09966, the entire contents of which is hereby incorpated by reference.

### Experiment 1: Induction of Antigen-specific Tolerance by OKTcdr4a in Cynomolgus Monkeys

### Tolerance measured by the HAMA response to OKTcdr4a

Since the HAMA response to the injected OKTcdr4a is easily measured, this immune response was used to determine whether some treatment protocols can induce immunological tolerance to OKTcdr4a itself and therefore might be useful clinically to induce tolerance to allografts and autoantigens.

Cynomolgus monkeys were injected with 0.2, 1, or 10 mg/kg/day ofOKTcdr4a, either on a single day or on 12 consecutive days. Although every monkey (n = 13) made an anti-antibody response to the CDR loops of OKTcdr4a, the two monkeys injected with 10 mg/kg/day of OKTcdr4a for 12 days (the highest dose tested) made the weakest and most transient anti-antibody responses (titers less than 1:1,500, which declined to background by day 40) (Table 1). One major difference observed in these animals was the length of time that the CD4 molecules on the peripheral blood CD4+ T cells remained saturated with OKTcdr4a. In this study with cynomolgus monkeys the critical time point was approximately 30-60 days post inital treatment.

Since immunological tolerance is a long term goal of anti-CD4 therapy, based on the early success obtained in rodent model systems (Shizuru et al.6, Cobbold et al.7) two animals that were treated with 10 mg/kg/day of OKTcdr4a for 12 days were further studied to determine whether antigen specific immunological tolerance in the human anti-mouse antibody (HAMA) response to OKTcdr4a had been induced. After the plasma levels of OKTcdr4a in these two monkeys had declined to <1 _g/ml, each was challenged with a single 10 mg/kg dose of OKTcdr4a. One animal made no anti-antibody response at all, while the second monkey made a weak, transient response that resembled its primary response. The two monkeys were then challenged 5-6 additional times with OKTcdr4a over a period of 600-700 days, and no significant anti-antibody responses were observed (Figures 1A and 2A). In contrast, three of the remaining 11 monkeys were similarly challenged with a single 10 mg/kg dose of OKTcdr4a, and all made typical strong secondary anti-antibody responses (mean peak titer of 1:157,000). The two animals treated with 10 mg/kg/day of OKTcdr4a for 12 days appeared to be specifically unresponsive to OKTcdr4a since they made normal antibody responses to an unrelated protein antigen, murine OKT4, (Figures 1B and 2B) and suffered no obvious immunodeficiency.

These results support the conclusion that the 10 mg/kg/day for 12 days protocol can induce a state of tolerance with regard to the HAMA response. Since both the murine and OKTcdr4a antibodies have higher affinities for human CD4 than for monkey CD4, lower doses and shorter dosing schedules may be sufficient to induce a similar state of tolerance in human patients.

### Experiment 2: Immunosuppression of Renal Allograft Rejection with OKTcdr4A in Cynomolgus Monkeys

In a previous study with OKTcdr4a in a non-human primate model of allograft rejection, the immunosuppressive activity of OKTcdr4a. This resulted in extended allograft survival times averaging 35 days (n=3), compared to 9 days in untreated recipients (n=4). However, a strong anti-antibody response was observed in each animal tested, associated with the loss of CD4 saturation, and long term immunological tolerance was not acheived in this model (Powelson et al. 1994). Long term immunological tolerance induction has also been demonstrated in a non-human primate model of renal allograft rejection with a pllyclonal rabbit anti-monkey thymocyte antiserum that depletes CD4= T cells from the peripheral blood, in combination with the administration of an HLA-DR (-/dim) fractioned cell population from donor bone marrow. (Thomas et al, Transplantation 51:198, 1991). With a protocol based on the previous study with rabbit anti-monkey thymocyte antiserum and donor bone marrow in the rhesus monkeu model, a study was designed to test whether OKTcdr4a could be substituted for the rabbit antiserum.

OKTcdr4a was administered intravenously to 13 rhesus monkey renal allograft recipients at a dose of 10 mg/kg/day for 21 days, beginning on the day of transplant, following partial MHC matching between the donor and recipient animals, (one shared allele at the DRB1 gene locus), as described previously (Thomas et al., 59:245-255, 1995). For 7 allograft recipients, donor bone marrow cells (Dr -/low) were prepared from bone marrow isolated from the allograft donor, as described previously (Thomas et al, Transplantation 51:198, 1991).

Four protocols were used, as shown in Table 2. (1) OKTcdr4a was used as the only immunosuppressive therapeutic (21 days, beginning on the day of transplant); (2) OKTcdr4a (21 days) was administered with the addition of donor bone marrow, on day 8 or day 9 post transplantation. (3) OKTcdr4a (21 days) was administered with the addition of cyclosporine 20 mg/kg p.o. on days 0-7 post transplant; (4) OKTcdr4a (21 days) was administered with the addition of cyclosporine 20 mg/kg orally on days 0-7 post transplant plus donor marrow, administered on day 8 or day 9 post transplantation.

OKTcdr4a treatment facilitated graft survival over 100 days in 5 out of the 13 recipients. Since long term graft survival times over 100 days were achieved in 3 out of 3 recipients using OKTcdr4a with donor bone marrow, OKTcdr4a treatment may establish a favorable milieu for tolerance induction with donor bone marrow cells.

Long term allograft survival also correlated with weak anti-antibody responses. Four of the 13 animals were strong responders with peak anti-antibody titers above 1:200,000 during dosing, resulting in reduced plasma levels of OKTcdr4a and reduced CD4 receptor occupancy. Three of these strong responders were cyclosporine-treated recipients. The anti-antibody response status also correlated with the anti-donor antibody response. None of the six low anti-antibody responders made detectable anti-donor antibody responses before 100 days, whereas three of the four high anti-antibody responders had positive anti-donor responses.

These studies suggest long term allograft survival and suppression of antidonor antibodies can be achieved, particularly after donor bone marrow infusion, by prolonged administration of OKTcdr4a to block CD4 receptors, provided the anti-antibody response is suppressed. This OKTcdr4a therapy appears to induce tolerance to OKTcdr4a, a process that may have been inhibited by a short course of cyclosporine. Concomitant suppression of anti-donor responses in low anti-antibody responders likely reflects inhibition of CD4-dependent T cell help to B cells by maintaining saturation of CD4 receptors.

**Table 1.**

| Anti-antibody responses to the "CDR loops" of OKTcdr4a and CD4 saturation in monkeys treated with OKTcdr4a. | | | | |
|---|---|---|---|---|
| Monkey ID | Protocol dose(mg/kg/d) × days | Duration of CD4 Saturation days >80% a | Primary Response 1 / titer | Secondary Respnse 1 / titer |
| 2124 | 0.2 × 1d | 0-1 | 10,000 | |
| 2138 | 0.2 × 1d | 0 | 6,000 | |
| 2102 | 1 × 1d | 0-1 | 7,000 | |
| 2116 | 1 × 1d | 2-3 | 18,000 | |
| 8521 | 10 × 1d | 8-11 | 6,000 | 70,000 |
| 2141 | 10 × 1d | 11-18 | 12,000 | |
| 2111 | 10 × 1d | 12-14 | 26,000 | |
| | 300,000 | | | |
| 8912 | 0.2 × 12d | 11-12 | 8,000 | 100,000 |
| F798 | 0.2 × 12d | 12-14 | 3,000 | |
| 8920 | 1 × 12d | 11-12 | 30,000 | |
| G551 | 1 × 12d | 11-12 | 85,000 | |
| G493 | 10 × 6d | 20-27 | 21,000 | |
| 8654 | 10 × 12d | 53-57 | 400 | 0 |
| G474 | 10 × 12d | 34-41 | 1,500 | 750 |

| | | | | |
|---|---|---|---|---|
| a Duration of saturation is indicated by the last day a sample was found to be 80% saturation and the first day a sample was found to be 80% saturation. | | | | |

**Table 2.**

| Graft survival, anti-antibody responses to the "CDR loops" antigen of OKTcdr4a and CD4 saturation in rhesus monkey renal allograft recipients treated with OKTcdr4a alone or in combination with cyclosporine A, and / or donor bone morrow. | | | | | |
|---|---|---|---|---|---|
| Monkey ID# | Protocol (mg/kg/d × d) ± DBM±CspA | Graft Survival days | Duration of Plasma >100µg/ml | Duration of CD4 Satur. >80% | Anti-mAb Response 1/titer |
| 8R2 | 10 × 21d | 170 | 84 | 100 | 1,000 |
| 91B078 | 10 × 21d | 23 | 16 | 15 | 700,000 |
| FOC | 10 × 21d | 54 | 54 | 54@ | >100 |
| 91B089 | 10 × 21d +DBM | >248 | 66 | 70 | 1,000 |
| 91B116 | 10 × 21d +DBM | 110 | 65 | 98 | 100 |
| F3F | 10 × 21d +DBM | >109 | >56 | 84 | 7,000 |
| Fw8 | 10 × 21d +CspA | 56 | 25 | 49 | 300,000 |
| 90096 | 10 × 21d +CspA | 31 | 20 | 15 | 3,000,000 |
| 99V | 10 × 21d +CspA+DBM | >475 | 53 | 84 | 20,000 |
| D7B | 10 × 21d +CspA+DBM | 66 | @65 | @65 | 1,000 |
| 90134 | 10 × 21d +CspA+DBM | 47 | @35 | 30 | 3,000,000 |
| AX | 10 × 21d +CspA+DMB | 33 | @33 | @33 | 10,000 |

| | | | | | |
|---|---|---|---|---|---|
| @ last sample tested at time of rejection | | | | | |

## Claims

1. A method of inducing immunological tolerance to an antigen in a primate subject comprising administering to the subject a combination of an amount of a non-depleting anti-CD4 antibody effective to induce immunological tolerance, and the antigen.

2. The method of claim 1 wherein the non-depleting anti-CD4 antibody is a humanized antibody.

3. The method of claim 2, wherein the non-depleting anti-CD4 antibody is a cdr-grafted antibody.

4. The method of claim 3, wherein the antibody is administered in an amount sufficient to maintain lymphocyte CD4 saturation for a sufficient period to permit induction of immunological tolerance.

5. The method of claim 4, wherein the cdr-grafted anti-CD4 antibody is administered in a dosage of about 0.5 mg/kg to about 10 mg/kg per day.

6. A method of inhibiting allograft rejection in a primate subject without depleting the subject's T-cells, comprising administering to the subject, at the time of the allograft, an amount of a non-depleting anti-CD4 antibody effective to inhibit allograft rejection.

7. The method of claim 6, wherein the non-depleting anti-CD4 antibody is a humanized antibody.

8. The method of claim 7, wherein the non-depleting anti-CD4 antibody is a cdr-grafted antibody.

9. The method of claim 8, wherein the antibody is administered in an amount sufficient to maintain lymphocyte CD4 saturation for a sufficient period to permit induction of immunological tolerance.

10. The method of claim 9, wherein the cdr-grafted anti-CD4 antibody is administered in a dosage of about 0.5 mg/kg to about 10 mg/kg.

11. The method of claim 6 further comprising administering donor bone marrow with the antibody.

12. A composition comprising an amount of a non-depleting anti-CD4 antibody effective to induce immunological tolerance, and a pharmacologically suitable carrier.

13. A composition of claim 12, wherein the non-depleting anti-CD4 antibody is a cdr-grafted antibody.

14. A composition of claim 13, wherein the effective amount is about 5 mg/kg.

15. A composition of claim 13, further donor bone marrow.
